# EUROPEAN PATENT APPLICATION

(11) **EP 4 193 996 A1**
(43) Date of publication of application: **14.06.2023**
(21) Application number: 21213919.0
(22) Date of filing: 11.12.2021
(51) Int. Cl.: A61K 31/513, A61K 31/704, A61K 45/06, A61K 9/00, A61P 31/20

(54) **GINSENOSIDES FOR TREATMENT OF CHRONIC HEPATITIS B VIRUS INFECTIONS**

(71) Applicant: Mendel University in Brno, 61300 Brno (CZ); Veterinary Research Institute, 62100 Brno (CZ)
(72) Inventor: Miller, Andrew David, London (GB); Ruzek, Daniel, Brno (CZ); Duraisamy, Ganesh Selvaraj, Brno (CZ); Adam, Vojtech, Brno (CZ); Heger, Zbynek, Brno (CZ)
(74) Representative: Harber IP s.r.o.

(57) **Abstract**

The present invention discloses ginsenosides used alone or in combination with nucleoside/nucleotide analogues for use in the treatment or prophylaxis of chronic hepatitis B virus infections.

## Description

### Field of Art

The present invention relates to ginsenosides for use in the treatment of chronic hepatitis B virus infections.

### Background Art

Ginsenosides are a class of natural product steroid glycosides and triterpene saponins found in *Panax ginseng.* Individual ginsenosides appear to exhibit a variety of biological effects, but these are often subtle and difficult to characterize in isolation [Attele, A.S; Wu, J.A; Yuan, C.S. Ginseng pharmacology: multiple constituents and multiple actions. Biochem. Pharmacol. 1999, 58, 1685-93]. Many studies suggest that ginsenosides have antioxidant properties, including free radical scavenging. Accordingly, ginsenosides have been suggested to be anti-proliferative with respect to cancer development and potentially neuroprotective with respect to Alzheimer's and Parkinson's diseases.

Chronic hepatitis B virus (HBV) infection remains a worldwide public health concern given its impact on the formation of severe liver diseases such as hepatocellular carcinoma.

The hepatitis B virus is herein abbreviated as HBV.

Currently, nucleoside/nucleotide analogue therapies, including treatment with lamivudine, telbivudine, entecavir, adefovir, or tenofovir, are approved for treatment of chronic HBV infection. However, nucleoside/nucleotide analogue treatments are not sufficient to achieve a functional cure, hence new active pharmaceutical ingredients are required.

### Disclosure of the Invention

The present invention relates to ginsenosides and mixtures thereof for use in treatment or prophylaxis of chronic HBV infections. Preferably the ginsenosides are selected from Rg6, Rh4 and Rb3.

The structural formulas of the said ginsenosides are as follows:

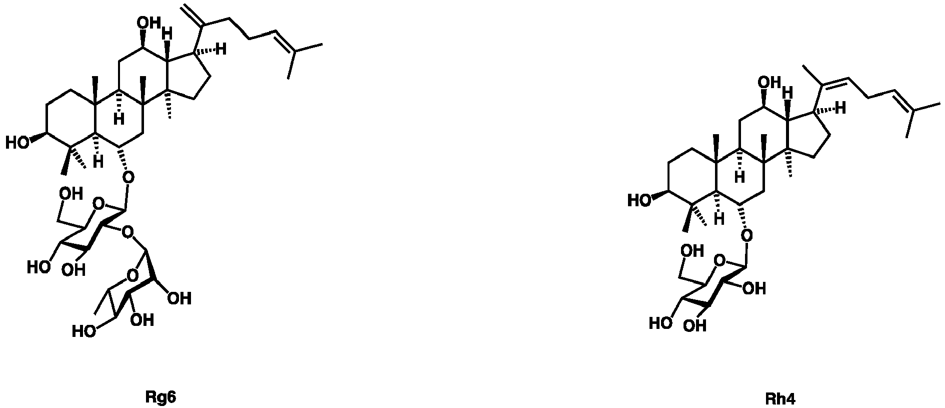

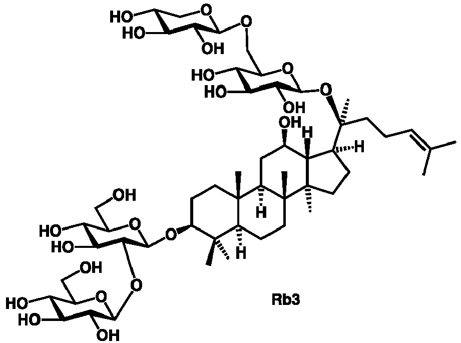

Within the framework of the present invention, the three ginsenosides Rg6, Rh4 and Rb3 were found unexpectedly to mediate efficient knockdown of hepatitis B surface (HBs) protein (S-HBs, M-HBs, and L-HBs) mRNA expression levels in the well-established *in vitro* recombinant hepatoma HBV cell model (HepG2.2.15) [Sells, M.A.; Chen, M.L.; Acs, G. Production of hepatitis B virus particles in Hep G2 cells transfected with cloned hepatitis B virus DNA. Proc. Natl. Acad. Sci. USA 1987, 84, 1005-1009, Wu, K.L; Zhang, X; Zhang, J; Yang, Y; Mu, Y.X; Liu, M; Lu, L; et al. Inhibition of Hepatitis B virus gene expression by single and dual small interfering RNA treatment. Virus Res. 2005, 112, 100-7]. In this context, Rg6, Rh4, and Rb3 mediate dose-dependent knockdowns of HBs protein mRNA expression levels with negative follow-on consequences for the levels of HBV DNA and HBs protein antigen (HBsAg) secretion. This "RNA interference surrogate" mechanism is a direct mechanism for the attenuation of HBV replication. At the same time, the ginsenosides show negligible cytotoxicity towards hepatic cells.

Furthermore, the present invention relates to a combination of at least one ginsenoside and at least one nucleoside/nucleotide analogue. Such combinations have a positive influence in the treatment or prophylaxis of chronic HBV infection. Preferably, ginsenosides mediate efficient knockdown of HBs protein mRNA expression levels by the "RNA interference surrogate" mechanism, and may be selected from Rg6, Rh4 or Rb3. Combinations involve doses of ginsenoside(s) at least 10x, 100x or 1000x (by molar ratio) higher than the doses of nucleoside/nucleotide analogue(s).

A particularly preferred combination is a combination of Rh4 and lamivudine (LMV). This combination exhibits synergism in the treatment or prophylaxis of chronic HBV infection. Hence, Rh4/lamivudine combinations are expected to reduce the required dose of lamivudine below the regulator approved dose in patients of 300 mg/per oral/daily.

The nucleoside/nucleotide analogue lamivudine is herein abbreviated as LMV.

The combination of at least one ginsenoside and at least one nucleoside/nucleotide analogue may be administered simultaneously or sequentially, according to the consideration of the clinician.

Nucleoside/nucleotide analogues are compounds containing a nucleic base analogue and a sugar (nucleoside analogues) or a nucleic base analogue, sugar and one to three phosphates (nucleotide analogues). They include, but are not limited to, LMV, telbivudine, entecavir, adefovir, adefovir dipivoxil, tenofovir, tenofovir disoproxil fumarate, clevudine, lagociclovir valactate, besifovir, tenofovir alafenamide, HDP-tenofovir, and AGX-1009 [Duraisamy, G.S; Bhosale, D; Lipenská, I; Huvarova, I; Růžek, D; Windisch, M.P; Miller, A.D. Advanced therapeutics, vaccinations, and precision medicine in the treatment and management of chronic hepatitis B viral infections; where are we and where are we going? Viruses 2020, 12, 998]. The term "treatment" refers to an approach for improving the health state of a patient. In particular, it involves alleviation of at least one symptom and/or diminishment of at least one symptom and/or preventing the worsening of at least one symptom, and/or decreasing the number of virus particles and virus-related antigens in the body of the patient, and/or decreasing the rate of replication of virus particles in the body of the patient.

The term "prophylaxis" refers to an approach for maintaining or improving the health state of a patient. In particular, it involves prevention of replication of virus and/or inhibition of a virus replication cycle. Prophylactic administration is typically performed before signs of the disease are detectable in the body of the patient.

The ginsenosides of the present invention, optionally together with nucleoside/nucleotide analogue(s), may be formulated in a pharmaceutical composition or drug delivery system. The pharmaceutical composition may be solid (e.g., tablets, coated tablets, soft capsules, hard capsules) or liquid (e.g., injection solution, infusion solution, solution for oral administration, emulsions or suspensions). The pharmaceutical compositions typically contain the active substance(s) and at least one pharmaceutically acceptable excipient. The excipients include solvents, diluents, binders, disintegrants, granulating agents, glidants, lubricants, coatings, colouring agents, preservatives, antioxidants, oils, emulsifiers. Drug delivery systems may be lipid-based nanoparticles, microemulsions, solid lipid nanoparticles, or transdermal patches.

### Brief Description of Drawings

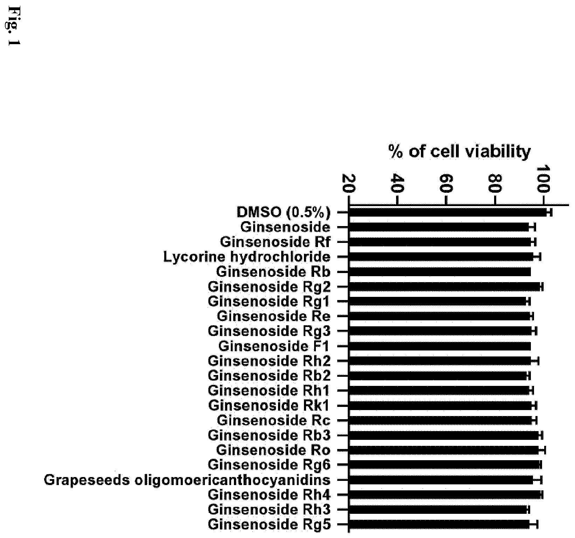

Fig. 1 Cell viabilities following exposure to ginsenoside library compounds. Ginsenoside library compounds were screened for their impact on cell viability. In this instance, HepG2.2.15 cells were treated with the indicated ginsenosides or other natural products (at 50 µM/well), then incubated. Using the cell counting kit-8 (CCK-8) assay, percentage cell viabilities were assessed relative to control cells [treated with dimethyl sulfoxide (DMSO) 0.5 % (v/v) alone] (100%), at day 4 post-first exposure to natural products. All data are represented as mean ± SD from experiments performed in duplicate (*p* < 0.05).

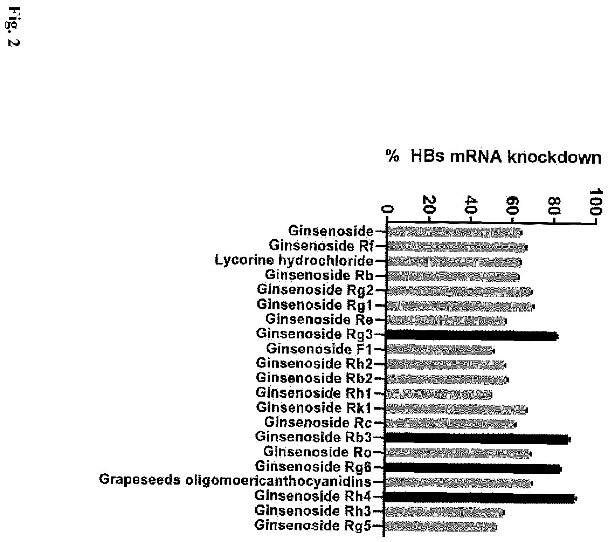

Fig. 2 Screening of ginsenoside library compound-mediated knockdown of HBs protein mRNA expression levels. HepG2.2.15 cells were treated with the indicated ginsenosides or other natural products (at 50 µM/well), then incubated. By means of reverse transcription quantitative polymerase chain reaction (RT-qPCR) analyses, percentage knockdowns of HBs protein mRNA expression levels were determined relative to glyceraldehyde 3-phosphate dehydrogenase (GAPDH) mRNA levels, at day 4 post-first exposure to natural products. All data are represented as mean ± SD from experiments performed in duplicate (*p* < 0.05).

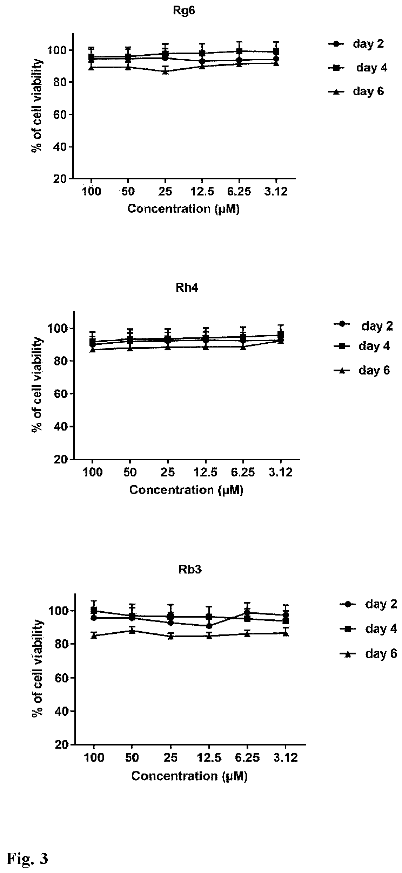

Fig. 3 Ginsenoside-mediated cytotoxicity effects. Selected ginsenosides (A-Rg6, B-Rh4, or C-Rb3) were studied with HepG2.2.15 cells. Cells were treated with the selected ginsenosides, at the indicated concentrations, then incubated. Using the CCK-8 assay, percentage cell viabilities were determined relative to control cells [treated with DMSO 0.5 % (v/v) alone] (100%), at day 2, 4 or 6 post-first exposure to ginsenosides. All data are represented as mean ± SD from experiments performed in triplicate (*p* < 0.05; *p* < 0.01).

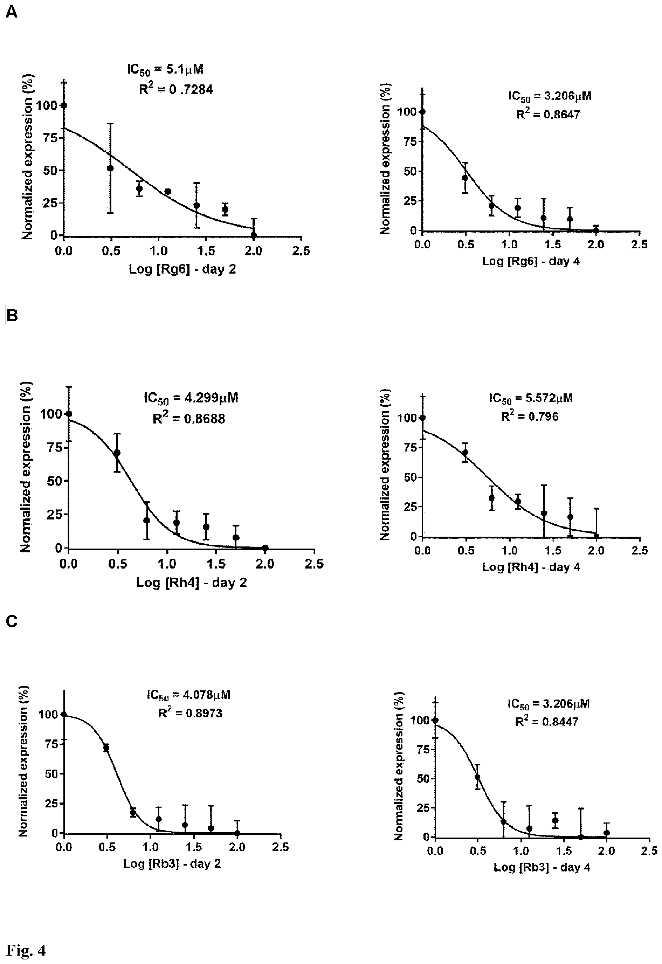

Fig. 4 Ginsenoside-mediated efficient knockdown of HBs protein mRNA expression levels. Selected ginsenosides (A-Rg6, B-Rh4, or C-Rb3) were studied with HepG2.2.15. Cells were treated separately with selected ginsenosides at different concentrations (3.1, 6.25, 12.5, 25, 50 or 100 µM), and control cells were treated with DMSO 0.5 % (v/v) alone. Following this, cells were incubated then normalized HBs protein mRNA expression levels were determined by RT-qPCR relative to GAPDH mRNA levels in cells, at day 2, 4, or 6 post-first exposure to ginsenosides or DMSO alone. Only day 2 and 4 data are shown.

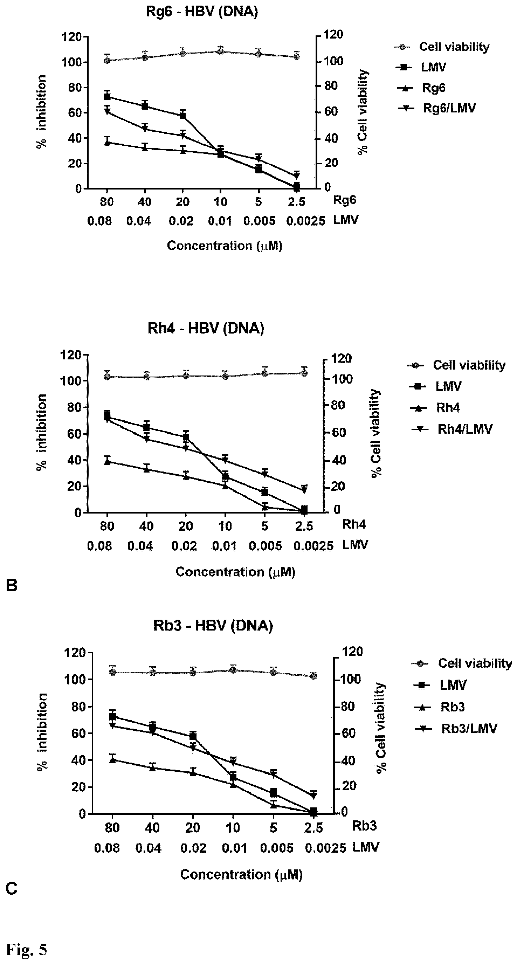

Fig. 5 Ginsenoside/LMV-mediated inhibition of HBV DNA secretion. Selected ginsenosides (A-Rg6, B-Rh4, or C-Rb3) were studied with HepG2.2.15 cells. Cells were initially treated with ginsenoside/LMV combinations, at the indicated concentrations, then incubated. Percentage cell viabilities were assessed relative to control cells [treated with DMSO 0.5 % (v/v) alone] (100%), at day 5 post-first exposure to ginsenoside/LMV combinations, using the CCK-8 assay. All data are represented as mean ± SD from experiments performed in duplicate (*p* < 0.05; *p* < 0.01). Alternatively, HepG2.2.15 cells were treated with LMV or ginsenosides, or ginsenoside/LMV combinations, at the indicated concentrations, then incubated. Following this, percentage inhibitions of HBV DNA secretion levels were determined by RT-qPCR relative to HBV-DNA levels secreted from control cells [treated with DMSO 0.5 % (v/v) alone] (100%), at day 5 post-first exposure. All data are represented as mean ± SD from experiments performed in triplicate (*p* < 0.05; *p* < 0.01).

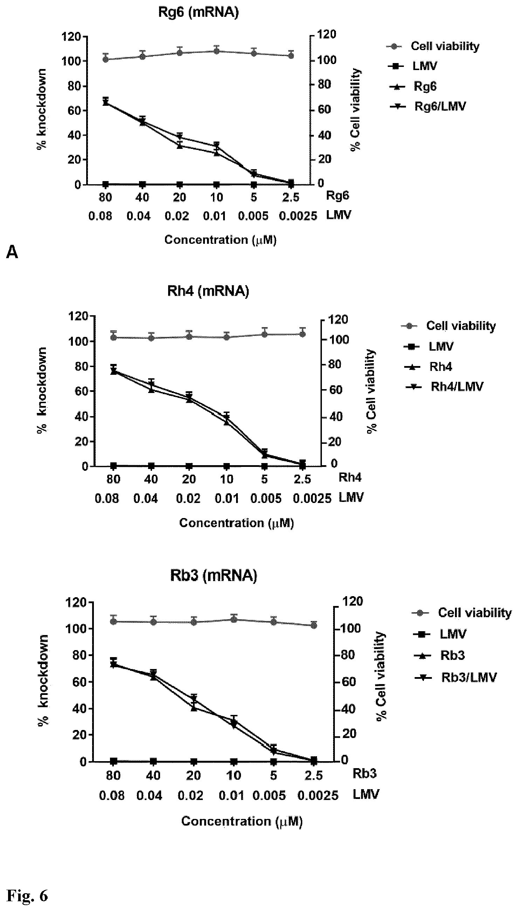

Fig. 6 Ginsenoside/LMV-mediated knockdown of HBs protein mRNA expression levels. Selected ginsenosides (A-Rg6, B-Rh4, or C-Rb3) were studied in HepG2.2.15 cells. Cells were initially treated with ginsenoside/LMV combinations, at the indicated concentrations, then incubated. Percentage cell viabilities were assessed relative to control cells [treated with DMSO 0.5 % (v/v) alone] (100%), at day 5 post-first exposure to ginsenoside/LMV combinations, using the CCK-8 assay. All data are represented as mean ± SD from experiments performed in duplicate *(p* < 0.05; *p <* 0.01). Alternatively, HepG2.2.15 cells were treated separately with LMV or ginsenosides, or ginsenoside/LMV combinations, at the indicated concentrations, then incubated. Thereafter, percentage knockdowns of HBs protein mRNA expression levels in cells were determined by means of RT-qPCR relative to GAPDH mRNA levels, at day 5 post-first exposure. All data are represented as mean ± SD from experiments performed in triplicate (*p* < 0.05; *p* < 0.01).

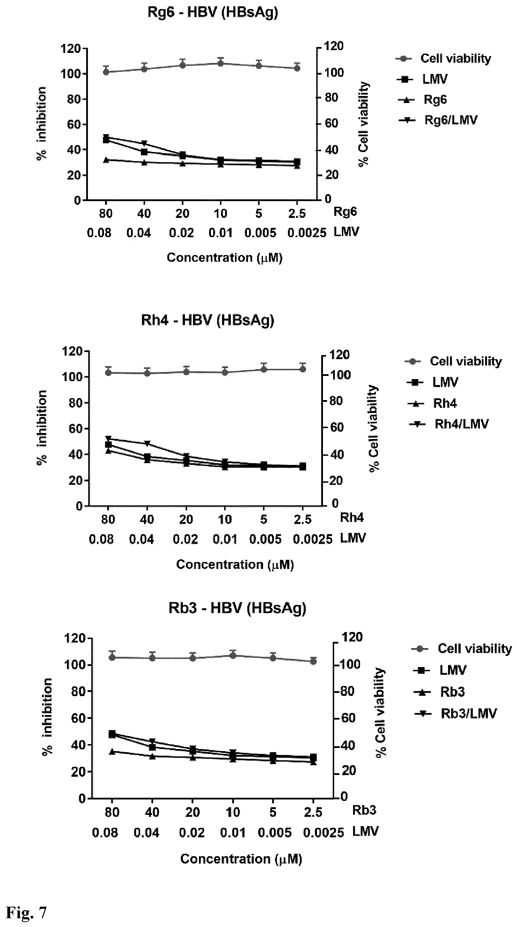

Fig. 7 Ginsenoside/LMV-mediated inhibition of HBsAg secretion. Selected ginsenosides (A-Rg6, B-Rh4, or C-Rb3) were studied in HepG2.2.15 cells. Cells were initially treated with ginsenoside/LMV combinations, at the indicated concentrations, then incubated. Percentage cell viabilities were assessed relative to control cells [treated with DMSO 0.5 % (v/v) alone] (100%), at day 5 post-first exposure to combinations, using the CCK-8 assay. All data are represented as mean ± SD from experiments performed in duplicate (*p* < 0.05; *p* < 0.01). Alternatively, cells were treated with LMV or ginsenosides, or ginsenoside/LMV combinations, at the indicated concentrations, then incubated. Percentage inhibitions of HBsAg secretion levels were determined by ELISA relative to HBsAg secretion levels from control cells [treated with DMSO 0.5 % (v/v) alone] (100%), at day 5 post-first exposure. All data are represented as mean ± SD from experiments performed in triplicate (*p* < 0.05; *p* < 0.01).

### Examples

### Material and Methods

### Natural compounds and materials

The human hepatoma cell line (HepG2.2.15) was a gift from Dr Jan Hodek (Virology Research-Service Team, Institute of Organic Chemistry and Biochemistry, Academy of Sciences of the Czech Republic). A ginsenoside library (20 compounds) was purchased from Sigma-Aldrich (St. Louis, MO, USA). Dulbecco's modified Eagle's medium (DMEM), fetal bovine serum (FBS) and an antibiotic-antimycotic cocktail were purchased from Biosera Inc (Philippines). LMV and antibiotics such as penicillin and streptomycin were also purchased from Sigma-Aldrich (St. Louis, MO, USA). The QuickTiter^{™} HBsAg ELISA kit for the detection of HBsAg was purchased from Cell Biolabs, Inc. (San Diego, CA, USA).

### Cell culture and selected ginsenosides

HepG2.2.15 cells were cultured at 37 °C, under 5 % CO₂, using DMEM culture medium supplemented with 10 % (v/v) FBS and an antibiotic-antimycotic cocktail (comprising penicillin 100 units/mL, streptomycin 100 µg/mL and G418 disulfate 250 µg/mL). Stock solutions (10 mM) of ginsenoside library compounds, ginsenosides (Rg6, Rh4, or Rb3) and LMV were prepared in DMSO and then diluted into various concentrations for *in vitro* experiments.

### Cytotoxicity assays

HepG2.2.15 cells (seeded at a density of 4 x 10⁴ cells per well in 96-well plates) were cultured at 37 °C in humidified air with 5 % CO₂. After cells reached 70-80 % confluence, initial screening of ginsenoside library compounds was performed with each compound added to cultured HepG2.2.15 cell at a final concentration of 50 µM. Cells were then further incubated at 37 °C under 5 % CO₂. At day 4 post-first exposure of cells to each ginsenoside library compound, percentage cell viabilities were determined by CCK-8 (Dojindo Laboratories, Kumamoto, Japan) used according to manufacturer's instructions. Thereafter, cytotoxicity studies were performed with appropriate quantities of selected ginsenosides (Rg6, Rh4, or Rb3) added to cultured HepG2.2.15 cells at final concentrations of 100, 50, 25, 12.5, 6.25 or 3.12 µM. Cells were then incubated at 37 °C under 5 % CO₂. At day 2, 4, and 6 post-first exposure of cells to the selected ginsenosides, percentage cell viabilities were determined by CCK-8 assay as above. Final cytotoxicity studies were performed with combinations of ginsenoside compounds (Rg6, Rh4 or Rb3) (at 80, 40, 20, 10, 5 or 2.5 µM) and LMV (at 0.08, 0.04, 0.02, 0.01, 0.005 or 0.0025 µM) respectively added to cultured HepG2.2.15 cells. Once more, cells were further incubated at 37 °C under 5 % CO₂. At day 5 post-first exposure of cells to ginsenoside/LMV combinations, percentage cell viabilities were determined by CCK-8 assay once again.

### HepG2.2.15 assays for HBs protein mRNA expression, HBV DNA and HBsAg secretion

HepG2.2.15 cells (seeded at a density of 4 x 10⁴ cells per well in 96-well plates) were cultured at 37 °C in humidified air with 5 % CO₂. Once cells had reached confluence, initial screening of ginsenoside library compounds was performed with each compound added individually to culture media at a final concentration of 50 µM. Cells were then further incubated at 37 °C under 5 % CO₂. At day 4 post-first exposure, cells were then collected to determine the percentage knockdowns of HBs protein mRNA expression levels relative to GAPDH mRNA levels (as described below).

Thereafter, more extensive studies were performed with HepG2.2.15 cells as follows. After cells were grown to confluence, as above, culture media were replaced with media containing one of the selected ginsenosides as appropriate, or else with LMV or ginsenoside/LMV combinations at the indicated concentrations. Cells were then further incubated at 37 °C under 5 % CO₂. At the indicated times (at day 2, 4, or 6 post-first exposure; or at just day 5 post-first exposure), cell and culture media samples respectively were collected for subsequent detection of HBs protein mRNA expression levels or secreted HBV DNA and HBsAg levels (as described below).

RNA extraction from cells was performed using the QIAamp^{®} Viral RNA Mini kit (Qiagen, Germany), according to manufacturer's instructions. RNA integrity was evaluated by electrophoresis on 1% agarose gels, and quantified using a ND1000 spectrophotometer (NanoDrop Technologies, Germany). Following this, extracted RNA (1 µg) was used to synthesize cDNA using a SensiFAST^{™} cDNA Synthesis Kit (Meridian Bioscience Inc, Cincinnati, OH, USA) according to manufacturer's instructions. RT-qPCR reactions were then performed with qPCR 2xSYBR master mix (Top-Bio, Czech Republic) on a LightCycler^{®} real-time PCR system (Roche, Switzerland), using the indicated primers specific for amplification of HBs protein (S-HBs, M-HBs, and L-HBs) and GAPDH control mRNAs (Table 1). PCR amplification was performed on multiple RT-qPCR reaction mixtures (each 20 µl) located in individual wells of a 96-well optical-grade PCR plate, sealed with optical sealing tape (Bio-Rad Laboratories, Hercules, CA, USA). Each RT-qPCR reaction mixture comprised synthesized cDNA (2 µl) (diluted 1:1), appropriate primers (each 0.4 µl, 10 mM), 2xSYBR green PCR master mix (10 µl) and ddH₂O (7.2 µl). PCR amplification involved one cycle at 94 °C (5 min), followed by 40 cycles of denaturation at 94 °C (20 s), then annealing and extension at 57 °C for 20 s, plus 72 °C for 30 s. Finally, melting curve analyses were performed by slowly heating the PCR reaction mixtures from 65 to 95 °C, in increments of 0.5 °C every 5 s, with simultaneous measurements of the SYBR green signal intensities. Percentage knockdowns of HBs protein mRNA expression levels were determined relative to GAPDH mRNA levels using calculated 2⁻ΔΔct values [Livak, K.J; Schmittgen, T.D. Analysis of relative gene expression data using real-time quantitative PCR and the 2(-Delta Delta C(T)) Method. Methods 2001, 25, 402-8]. Three biological replicates were performed for each data point where fold change (FC) ≥ 2 is considered statistically significant.

**Table 1. Primers for RT-qPCR detection of specific mRNAs**

| **mRNA** | **Primers** | **Accession Number** |
|---|---|---|
| HBs-Fr | CCTAGGACCCCTGCTCGTGT (SEQ ID NO:1) | M38454.1 |
| HBs-Rv | AACGCCGCAGACACATCCAA (SEQ ID NO:2) | M38454.1 |
| GADPH-Fr | CGGATTTGGTCGTATTGGG (SEQ ID NO:3) | J02642.1 |
| GADPH-Rv | TCTCGCTCCTGGAAGATGG (SEQ ID NO:4) | J02642.1 |

HBV DNA was isolated from HepG2.2.15 culture media samples by means of the QIAGEN QIAamp DNA mini kit (Qiagen, Hilden, Germany), used according to manufacturer's instructions. Percentage inhibitions of HBV DNA secretion levels were determined in HepG2.2.15 culture media samples by RT-qPCR relative to HBV-DNA levels secreted from control cells [treated with DMSO 0.5 % (v/v) alone] (100%). RT-qPCR reactions were performed with the Luna^{®} Universal Probe qPCR master mix (New England Biolabs, Ipswich, MA, USA) on a LightCycler^{®} real-time PCR system (Roche, Switzerland), using the indicated primers (Table 2). PCR amplification involved initial denaturation at 95 °C (60 s), followed by 45 cycles at 95 °C (15 s), then a single cycle at 60 °C (30 s).

Percentage inhibitions of HBsAg secretion levels were determined in HepG2.2.15 culture media samples by ELISA relative to HBsAg secretion levels from control cells [treated with DMSO 0.5 % (v/v) alone] (100%). ELISA reactions were performed with a commercial ELISA assay kit (Cell Biolabs, Inc., San Diego, CA) according to manufacturer's instructions, wherein quantification of HBsAg levels was performed by measuring optical density (OD) values at 450/630nm using a microtiter plate spectrophotometer.

**Table 2. Primers for RT-qPCR detection of HBV DNA**

| **DNA** | **Primers** | **Accession Number** |
|---|---|---|
| HBV-Fr | ACTCACCAACCTCCTGTCCT (SEQ ID NO:5) | X02763.1 |
| HBV-Rv | GACAAACGGGCAACATACCT (SEQ ID NO:6) | X02763.1 |
| DNA probe | 5'- FAM-TATCGCTGGATGTGTCTGCGGCGT-3'-TAMRA | X02763.1 |
| | (SEQ ID NO:7) | |

### Determination of ginsenoside/LMV combination indices

The effects of ginsenoside/LMV combinations were analyzed using the Chou-Talalay method. The combination index (CI) values of drug combinations were calculated using CompuSyn software (CompuSyn Inc., Paramus, NJ). CI values of > 1.10, 0.9 to 1.10, and < 0.9 to 0.3 indicated if drug combinations were antagonistic, additive, or synergistic, respectively [Chou T.C. Theoretical basis, experimental design, and computerized simulation of synergism and antagonism in drug combination studies. Pharmacol. Re. 2006, 58, 621-681, Chou, T.C. Drug combination studies and their synergy quantification using the Chou-Talalay method. Cancer Res. 2010, 70, 440-6].

### Statistical Analysis

All experiments were performed in triplicate, unless otherwise indicated in the figures, and data analyzed by GraphPad Prism 7 (Graph Pad Software, Inc., USA). Results are expressed as mean +/- standard deviation (SD). Statistical comparisons were made using the Holm-Sidak multiple t-test adjusted for multiplicity.

### Results

### Screening a ginsenoside compound library for anti-HBV activity

Initially, a ginsenoside library was screened against HepG2.2.15 cells for cytotoxic effects at a high but not excessive concentration. At day 4 post-first exposure to each member of the ginsenoside library (at 50 µM), HepG2.2.15 cell viabilities were measured by CCK-8 assay. The ginsenoside library compounds did not cause significant cytotoxicity compared to the situation with control cells [treated with DMSO 0.5 % (v/v) alone] (Fig. 1). Thereafter, the ginsenoside library compounds were screened for their ability to knockdown HBs protein mRNA expression levels in HepG2.2.15 cells. At day 4 post-first exposure to each individual member of the ginsenoside library (at 50 µM), percentage knockdowns of HBs protein mRNA expression levels were determined by RT-qPCR. All members of the ginsenoside library appeared able to knockdown HBs protein mRNA expression levels in HepG2.2.15 cells > 50%. However, ginsenosides Rg6, Rh4, Rb3, and Rg3 were shown to be especially effective (Fig. 2). The activity of Rg3 against HBV has been noted previously [Kang, L. J; Choi, Y. J; Lee, S. G. Stimulation of TRAF6/TAK1 degradation and inhibition of JNK/AP-1 signalling by ginsenoside Rg3 attenuates hepatitis B virus replication. Int. J. Biochem. Cell Biol. 2013, 45, 2612-21]. Accordingly, only ginsenosides Rg6, Rh4, and Rb3, were selected for further investigation.

### Evaluation of cytotoxicity effects of selected ginsenosides

The HepG2.2.15 cytotoxicities of all three selected ginsenosides (Rg6, Rh4, or Rb3) were analyzed in a dose-dependent manner, at day 2, 4, and 6 post-first exposure to the ginsenosides, using the CCK-8 assay once more. All three selected ginsenosides showed no significant HepG2.2.15 cytotoxicity at day 2 and 4, post-first exposure. Slightly reduced cell viabilities were observed in general at day 6 post-first exposure in a manner unrelated to ginsenoside doses, so indicative of a non-specific decline in HepG2.2.15 cell viability after 6 days of cell culture compared to control cells [treated with DMSO 0.5 % (v/v) alone] (Fig. 3).

### Efficient knockdown of HBs protein mRNA expression levels by selected ginsenosides

HepG2.2.15 cells were treated with each of the selected ginsenosides indicated concentrations to observe efficient, dose-dependent knockdowns of HBs protein mRNA expression levels relative to control GAPDH mRNA levels in HepG2.2.15 cells (Fig. 4). Efficient knockdowns of intracellular HBs protein mRNA expression levels were observed at day 2 and 4, post-first exposure to ginsenosides Rg6, Rh4, and Rb3. However, these knockdown effects were observed to be transient and to wear off completely by day 6, post-first exposure (data not shown). Estimated ICso values for ginsenosides Rg6, Rh4 and Rb3 were 5.1, 4.3, and 4.1 at day 2, and 3.2, 5.6, and 3.2 µM at day 4, respectively, post-first exposure of HepG2.2.15 cells. All three of the selected ginsenosides behaved similarly in mediating efficient knockdowns of HBs protein mRNA expression levels in HepG2.2.15 cells. Critically, our data are also unambiguous in suggesting that ginsenosides Rg6, Rh4, and Rb3 are potentially able to modulate the HBV replication cycle by acting during and/or post-viral mRNA transcription, in a manner that appears analogous to RNA interference (RNAi) effects as seen using traditional effectors such as small interfering RNAs (siRNAs) [Duraisamy, G.S; Bhosale, D; Lipenská, I; Huvarova, I; Růžek, D; Windisch, M.P; Miller, A.D. Advanced therapeutics, vaccinations, and precision medicine in the treatment and management of chronic hepatitis B viral infections; where are we and where are we going? Viruses 2020, 12, 998]. The mechanism of action Rg3 against the HBV replication cycle has been previously described as being altogether different [Kang, L. J; Choi, Y. J; Lee, S. G. Stimulation of TRAF6/TAK1 degradation and inhibition of JNK/AP-1 signalling by ginsenoside Rg3 attenuates hepatitis B virus replication. Int. J. Biochem. Cell Biol. 2013, 45, 2612-21].

### Ginsenosides inhibit secretion of HBV DNA from HepG2.2.15 cells

Next, we undertook to study the impact of the selected ginsenoside compounds on the levels of secretion of HBV DNA from HepG2.2.15 cells in the presence and absence of LMV. Initially, the cytoxicities of ginsenoside/LMV combinations were studied in HepG2.2.15 cells, using CCK-8 assays. Data indicated that HepG2.2.15 cells were largely refractory to the presence of up to 80 µM of Rg6, Rh4 or Rb3 in combination with up to 0.08 µM of LMV (Fig. 5). Thereafter, the respective impacts of LMV alone, ginsenosides alone, or ginsenoside/LMV combinations on the percentage inhibition of secretion of HBV DNA secretion were evaluated. As anticipated, LMV alone significantly inhibited the levels of secreted HBV DNA, the selected ginsenosides much less so (Fig. 5). Based on the available data, CI values were then computed, by the Chou-Talalay method [Chou T.C. Theoretical basis, experimental design, and computerized simulation of synergism and antagonism in drug combination studies. Pharmacol. Re. 2006, 58, 621-681, Chou, T.C. Drug combination studies and their synergy quantification using the Chou-Talalay method. Cancer Res. 2010, 70, 440-6], looking for potential ginsenoside/LMV synergistic, additive, or antagonistic effects (Table 3).

**Table 3. Combination Indices - for inhibition of HBV DNA secretion**

| **Drug combination** | **Ratio** | **CI values for modulation of HBV DNA levels** | | | | **Weighted average CI values** | **Assigned symbol** | **Description** |
|---|---|---|---|---|---|---|---|---|
| | | **50%** | **75%** | **90%** | **95%** | | | |
| Rg6/LMV | 1000:1 | 1.2045 | 1.1976 | 1.1868 | 1.0827 | 1.18 | - | Slight antagonism |
| Rh4/LMV | 1000:1 | 1.0617 | 0.9231 | 0.8316 | 0.8219 | 0.88 | + | Slight synergism |
| Rb3/LMV | 1000:1 | 1.0865 | 0.9636 | 0.9032 | 0.8334 | 0.98 | ± | Near additive |

These results confirmed that whilst LMV has a direct impact on secreted HBV DNA levels, Rh4/LMV combinations acted synergistically to inhibit HBV DNA secretion, but Rg6/LMV combinations acted antagonistically. Generally speaking, ginsenoside/LMV combinations were most evidently beneficial at ginsenoside concentrations of 10 µM or below, with the opposite appearing true at higher ginsenoside concentrations. Conversely and unsurprisingly, LMV was found to have no obvious impact upon HBs protein mRNA expression levels in stark contrast to the selected ginsenosides (Fig. 6).

### Ginsenosides inhibit secretion of HBsAg from HepG2.2.15 cells

Turning to the effects of the selected ginsenosides on HBsAg secretion, HepG2.2.15 cells were treated with LMV alone, ginsenosides alone or various ginsenoside/LMV combinations, as above, and the impacts on inhibition of HBsAg secretion were evaluated. Importantly, significant reductions in HBsAg secretion were observed with the selected ginsenosides alone and generally more so with LMV alone. However, ginsenoside/LMV combinations were the most effective (Fig. 7). Indeed, a maximum reduction of 52% in secreted HBsAg levels was reached when HepG2.2.15 cells were treated with the Rh4/LMV combination (at respective drug concentrations of 80 µM and 0.08 µM) compared to the situation with control HepG2.2.15 cells. The Rh4/LMV combination appeared to be more effective than either the Rg6/LMV or Rb3/LMV combinations. Once again, based on the available data, CI values were then computed, by the Chou-Talalay method [Chou T.C. Theoretical basis, experimental design, and computerized simulation of synergism and antagonism in drug combination studies. Pharmacol. Re. 2006, 58, 621-681, Chou, T.C. Drug combination studies and their synergy quantification using the Chou-Talalay method. Cancer Res. 2010, 70, 440-6] looking for potential ginsenoside/LMV synergistic, additive, or antagonistic effects (Table 4).

**Table 4. Combination Indices - for inhibition of HBsAg secretion**

| **Drug combination** | **Ratio** | **CI values at inhibition of HBsAg secretion** | | | | **Weighted average CI values** | **Assigned symbol** | **Description** |
|---|---|---|---|---|---|---|---|---|
| | | **50%** | **75%** | **90%** | **95%** | | | |
| Rg6/LMV | 1000:1 | 1.6218 | 1.3533 | 1.2869 | 0.7847 | 1.39 | -- | Moderate antagonism |
| Rh4/LMV | 1000:1 | 1.6511 | 0.9757 | 0.7208 | 0.5200 | 0.90 | + | Slight synerigsm |
| Rb3/LMV | 1000:1 | 1.1234 | 1.0565 | 0.9229 | 0.7594 | 1.07 | ± | Near additive |

## Claims

1. Ginsenoside selected from Rg6, Rh4, Rb3 and mixtures thereof for use in treatment or prophylaxis of chronic hepatitis B virus infections.

2. Ginsenoside for use according to claim 1, wherein the chronic hepatitis B virus infections are responsive to knockdown of HBs protein mRNA expression levels.

3. Combination of at least one ginsenoside and at least one nucleoside/nucleotide analogue for use in treatment or prophylaxis of chronic hepatitis B virus infections.

4. Combination for use according to claim 3 wherein the molar ratio of the at least one ginsenoside to the at least one nucleoside/nucleotide analogue is at least 1000:1.

5. Combination for use according to claim 3 wherein the molar ratio of the at least one ginsenoside to the at least one nucleoside/nucleotide analogue is at least 100:1.

6. Combination for use according to claim 3 wherein the molar ratio of the at least one ginsenoside to the at least one nucleoside/nucleotide analogue is at least 10:1.

7. Combination for use according to any one of claims 3 to 6 wherein the chronic hepatitis B virus infections are responsive to knockdown of HBs protein mRNA expression levels.

8. Combination for use according to any one of claims 3 to 7 wherein the at least one ginsenoside is administered simultaneously with the at least one nucleoside/nucleotide analogue.

9. Combination for use according to any one of claims 3 to 7 wherein the at least one ginsenoside and the at least one nucleoside/nucleotide analogue are administered sequentially.

10. Combination for use according to any one of claims 3 to 9 wherein the ginsenoside is selected from Rg6, Rh4 and Rb3.

11. Combination for use according to any one of claims 3 to 9 wherein the ginsenoside is Rh4 and the nucleoside/nucleotide analogue is lamivudine.

12. A pharmaceutical composition comprising at least one ginsenoside and at least one nucleoside/nucleotide analogue as active ingredients, and optionally further at least one pharmaceutically acceptable excipient and/or drug delivery system.

13. The pharmaceutical composition according to claim 12 wherein the ginsenoside is selected from Rg6, Rh4 and Rb3, and/or the nucleoside/nucleotide analogue is lamivudine.

14. The pharmaceutical composition according to claim 12, wherein the ginsenoside is Rh4 and the nucleoside/nucleotide analogue is lamivudine.
